# EUROPEAN PATENT SPECIFICATION

(11) **EP 0 923 942 B1**
(45) Date of publication and mention of the grant of the patent: **14.11.2001**
(21) Application number: 97901835.5
(22) Date of filing: 04.02.1997
(51) Int. Cl.: A61K 47/30, A61K 31/58, A61K 9/14, A61K 47/32, A61K 47/38

(54) **5alpha-REDUCTASE INHIBITORY PREPARATION FOR ORAL ADMINISTRATION, PROCESS FOR PRODUCING THE SAME, AND USE THEREOF**
5-alpha REDUCTASE INHIBIERENDE ZUSAMMENSTELLUNG ZUR ORALEN VERABREICHUNG, VERFAHREN ZUR HERSTELLUNG UND DEREN VERWENDUNG
PREPARATION INHIBITRICE DE LA 5alpha-REDUCTASE ADMINISTRABLE PAR VOIE ORALE, PROCEDE DE PRODUCTION ET UTILISATION

(30) Priority: 05.02.1996 JP 1871896
(43) Date of publication of application: 23.06.1999
(73) Proprietor: Sankyo Company, Limited, Tokyo 103-8426 (JP)
(72) Inventor: USUI, Fusao, Sankyo Company, Ltd., Tokyo 140 (JP); OHUCHI, Yuko, Sankyo Company , Ltd., Tokyo 140 (JP); KUSAI, Akira, Sankyo Company , Ltd., Tokyo 140 (JP)
(74) Representative: Gibson, Christian John Robert
(86) International application number: JP9700273
(87) International publication number: WO9727875

(56) References cited:
- GB-A- 2 153 678
- JP-A- 5 032 693
- JP-A- 60 222 497
- CHEMICAL ABSTRACTS, vol. 122, no. 15, 10 April 1995 Columbus, Ohio, US; abstract no. 187869, XP002095003 & JP 06 293793 A (SANKYO) 21 October 1994

## Description

The present invention relates to a formulation of a 5α-reductase inhibitor for oral administration, and a preparation process and use thereof.

### Background Art

The azasteroids included as an active ingredient in the formulation of a 5α-reductase inhibitor for oral administration of the present invention, namely, N-[1-(4-methoxyphenyl)-1-methylethyl]-3-oxo-4-aza-5α-androst-1-ene-17β-carboxamide [the compound represented by the formula (I) shown below] and N-(1,1-dimethylethyl)-3-oxo-4-aza-5a-androst-1-ene-17β-carboxamide [the compound represented by the formula (II) shown below] are known compounds and exhibit 5α-reductase inhibitory action. These compounds are therefore useful as a preventive and/or therapeutic agent for prostatic hypertrophy.

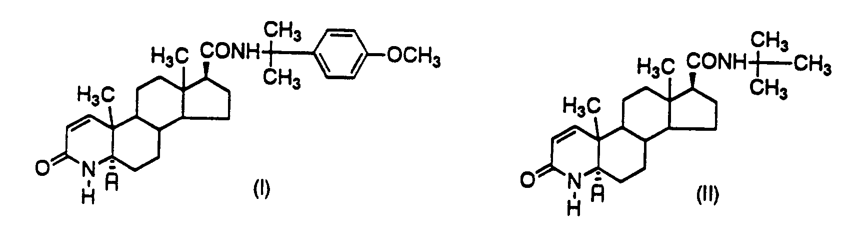

However, the above-described compounds have poor water solubility and it has so far been difficult to prepare a solid formulation containing such a compound because of the following reasons.

When a pharmaceutical formulation is prepared from a sparingly-water-soluble compound in a manner known per se in the art, the resulting formulation generally has a low dissolution property and therefore does not exhibit sufficient bioavailability. The present inventors practically prepared a pharmaceutical formulation from the above-described azasteroid in a manner known per se in the art, but the resulting formulation had a markedly low dissolution property.

In such a case, the method of adding an excess amount of an active ingredient in one dosage unit is thought as a countermeasure for providing sufficient bioavailability for the formulation. Such a method is however inefficient because the preparation of a pharmaceutical formulation containing an excess amount of an active ingredient requires an excess amount of the active ingredient. In addition, the administration of such a formulation containing an excess of active ingredient causes fluctuations of the absorption ratio among individual living bodies administered, which heightens the possibility of causing undesirable side effects. The above method cannot therefore be put into practical use.

Concerning the formulation of such a sparingly-water-soluble compound, various techniques for improving its dissolution property have so far been reported. Problems such as what is a suitable method for improving the dissolution property of a specific compound and how much the dissolution property can be improved depend on various physical properties (for example, crystal form and particle size) and chemical properties (for example, kind and number of the functional groups). As a matter of fact, these problems will be solved for the first time by preparing various formulations containing the compound in accordance with various processes and evaluating the resulting formulations in practice.

A method of subjecting a compound serving as an active ingredient and a pharmacologically acceptable ingredient to mixed-grinding is one technique for improving the dissolution property. Concerning this technique, reports have been made as follows:

The fact that in the case where a mixture of phenytoin and casein sodium is ground and in the case where a mixture of phenytoin, casein sodium and oleic acid is ground, the solubility of phenytoin increases, while in the case where a mixture of phenytoin, casein sodium and palmitic acid is ground, no change occurs in the solubility is disclosed in "YAKUZAIGAKU", 50(2), 187-192(1990)",

In "YAKUZAIGAKU", 49(1), 70-77(1989)", it is disclosed that grinding a mixture of 9,3"-diacetylmidecamycin with polyvinylpyrrolidone yields amorphous 9,3"-diacetylmidecamycin.

In "Japanese Patent Application Kokai No. SHO 60-181030 (GB-A-2153678)", there is disclosed a pharmaceutical formulation having an improved dissolution property obtained by grinding a mixture of a synthesized steroid (Medroxyprogesterone) with cross-linked polyvinylpyrrolidone (crospovidone).

The present inventor therefore ground a mixture of the above azasteroid with crospovidone in accordance with the process disclosed in the above Japanese Patent Application Kokai No. SHO 60-181030 in which a compound having a relatively similar structure to the azasteroid of the present invention is disclosed, but a composition having a dissolution property sufficiently improved for practical use could not be obtained.

Described specifically, the kind of ingredient and mixing ratio thereof suitable for improving the dissolution property of an active ingredient by grinding a mixture thereof depends on the physical and chemical properties of the active ingredient. Whether a process according to the prior art will bring about a similar effect or not does not depend simply on the similarity or non-similarity of the chemical structure of the active ingredient.

The present inventors therefore carried out a further investigation on a process for improving the dissolution property of the above-described azasteroid with a view to providing its formulation for practical use.

As a result, in the present invention, it has been found that the constitution of the present invention brings about a marked improvement in the dissolution property. In "YAKUGAKU ZASSHI, 109(12), 932-937(1989)", there is disclosed a similar composition except for the compound contained therein as the active ingredient. However, the marked improvement in the dissolution property brought about by the present invention cannot be expected from the above prior art. According to the present invention, an orally-dosable solid composition of the above azasteroid, which has so far been unsuitable for practical use in an orally-dosable solid composition, can be obtained having a dissolution property which, for the first time, is sufficient for practical use.

### Disclosure of the Invention

The present inventors have carried out an extensive investigation on the formulation of 5α-reductase inhibitors for oral administration containing an azasteroid. As a result, it has been found that the dissolution property of the azasteroid can be markedly improved by appropriately selecting pharmacologically acceptable ingredients to be used in combination and carrying out grinding of a mixture of these ingredients and the azasteroid, leading to the completion of the present invention.

In one aspect of the present invention;
(1) There is thus provided a formulation of a 5α-reductase inhibitor for oral administration which comprises a composition obtained by grinding a mixture containing one azasteroid selected from the group (A) shown below, a water-soluble polymer and a disintegrant:
   Group (A)
   N-[1-(4-methoxyphenyl)-1-methylethyl]-3-oxo-4-aza-5α-androst-1-ene-17β-carboxamide; and
   N-(1,1-dimethylethyl)-3-oxo-4-aza-5α-androst-1-ene-17β-carboxamide.

   Preferred are:
(2) a formulation of a 5α-reductase inhibitor for oral administration as described above, wherein said one azasteroid selected from the group (A) is N-[1-(4-methoxyphenyl)-1-methylethyl]-3-oxo-4-aza-5α-androst-1-ene-17β-carboxamide;
(3) a formulation of a 5α-reductase inhibitor for oral administration as described above, wherein said water-soluble polymer is a water-soluble cellulose derivative or polyalkenylpyrrolidone derivative;
(4) a formulation of a 5α-reductase inhibitor for oral administration as described above, wherein said water-soluble polymer is hydroxypropylmethylcellulose, hydroxypropylcellulose or polyvinylpyrrolidone;
(5) a formulation of a 5α-reductase inhibitor for oral administration as described above, wherein said disintegrant is sodium carboxymethyl starch, crospovidone, carmellose, carmellose calcium, croscarmellose sodium, low-substituted hydroxypropylcellulose or crystalline cellulose;
(6) a formulation of a 5α-reductase inhibitor for oral administration as described above, wherein said disintegrant is sodium carboxymethyl starch, crospovidone, carmellose, carmellose calcium or croscarmellose sodium;
(7) a formulation of a 5α-reductase inhibitor for oral administration as described above, wherein said water-soluble polymer is added in an amount of 0.1 to 500 parts by weight based on 1 part by weight of said azasteroid;
(8) a formulation of a 5α-reductase inhibitor for oral administration as described above, wherein said disintegrant is added in an amount of 0.1 to 500 parts by weight based on 1 part by weight of said azasteroid; and
(9) a formulation of a 5α-reductase inhibitor for oral administration as described above, wherein said disintegrant is added in an amount of 0.5 to 10 parts by weight based on 1 part by weight of said water-soluble polymer.
   In another aspect of the present invention, there is also provided:
(10) A process for the preparation of a formulation of a 5α-reductase inhibitor for oral administration, which comprises grinding a mixture containing one azasteroid selected from the group (A) shown below, a water-soluble polymer and a disintegrant:
   Group A
   N-[1-(4-methoxyphenyl)-1-methylethyl]-3-oxo-4-aza-5α-androst-1-ene-17β-carboxamide; and
   N-(1,1-dimethylethyl)-3-oxo-4-aza-5α-androst-1-ene-17β-carboxamide.

   Preferred are:
(11) A process as described above, wherein said one azasteroid selected from the group (A) is N-[1-(4-methoxyphenyl)-1-methylethyl]-3-oxo-4-aza-5α-androst-1-ene-17β-carboxamide;
(12) A process as described above, wherein said water-soluble polymer is a water-soluble cellulose derivative or a polyalkenylpyrrolidone derivative;
(13) A process as described above, wherein said water-soluble polymer is hydroxypropylmethylcellulose, hydroxypropylcellulose or polyvinylpyrrolidone;
(14) A process as described above, wherein said disintegrant is sodium carboxymethyl starch, crospovidone, carmellose, carmellose calcium, croscarmellose sodium, low-substituted hydroxypropylcellulose or crystalline cellulose;
(15) A process as described above, wherein said disintegrant is carboxymethyl starch sodium, crospovidone, carmellose, carmellose calcium or croscarmellose sodium;
(16) A process as described above, wherein said water-soluble polymer is added in an amount of 0.1 to 500 parts by weight based on 1 part by weight of said azasteroid;
(17) A process as described above, wherein said disintegrant is added in an amount of 0.1 to 500 parts by weight based on 1 part by weight of said azasteroid; and
(18) A process as described above, wherein said disintegrant is added in an amount of 0.5 to 10 parts by weight based on 1 part by weight of said water-soluble polymer.

In the "formulation of a 5α-reductase inhibitor for oral administration" according to the present invention, the term "azasteroid" means N-[1-(4-methoxyphenyl)-1-methylethyl]-3-oxo-4-aza-5α-androst-1-ene-17β-carboxamide or N-(1,1-dimethylethyl)-3-oxo-4-aza-5α-androst-1-ene-17β-carboxamide. Preferred is N-[1-(4-methoxyphenyl)-1-methylethyl]-3-oxo-4-aza-5α-androst-1-ene-17β-carboxamide.

The above compounds happen to absorb water, thereby having adsorbed water or becoming hydrates when allowed to stand in the air or during recrystallization. Such hydrates are also embraced in the "azasteroid".

As the "water-soluble polymer" and "disintegrant" contained in the ground mixture for obtaining the "formulation of a 5α-reductase inhibitor for oral administration" of the present invention, the following compounds can be given as examples.

Examples of the "water-soluble polymer" include water-soluble cellulose derivatives such as hydroxypropylmethylcellulose, hydroxypropylcellulose, hydroxyethylcellulose, carmellose sodium and methylcellulose; polyalkenylpyrrolidone derivatives such as polyvinylpyrrolidone; polyalkenyl alcohol derivatives such as polyvinyl alcohol; carboxyalkenyl polymer derivatives such as carboxyvinyl polymer; polyalkenyl organic acid derivatives such as partially-saponified polyvinyl acetate; alkenyl organic acid ester copolymer derivatives such as vinyl acetate copolymer; salts of a polysaccharide such as sodium arginate; ester derivatives of a polysaccharide such as propylene glycol arginate; and polyalkylene glycol derivatives such as polyethylene glycol. Among them, preferred are water-soluble cellulose derivatives and polyalkenylpyrrolidone derivatives, and hydroxypropylmethylcellulose, hydroxypropylcellulose and polyvinylpyrrolidone are more preferred. These water-soluble polymers can be used either singly or in combination.

Examples of the "disintegrant" include starch, partially α-converted starch, carboxymethyl starch, hydroxypropyl starch, sodium carboxymethyl starch, crospovidone, carmellose, carmellose calcium, croscarmellose sodium and low-substituted hydroxypropylcellulose and water-insoluble cellulose derivatives (e.g. crystalline cellulose, ethylcellulose, cellulose acetate, carboxymethylethylcellulose and nitrocellulose). Among them, preferred are sodium carboxymethyl starch, crospovidone, carmellose, carmellose calcium, croscarmellose sodium, low-substituted hydroxypropylcellulose and crystalline cellulose, and sodium carboxymethyl starch, crospovidone, carmellose, carmellose calcium and croscarmellose sodium are more preferred. The above-exemplified disintegrants can be used either singly or in combination.

The above-described water-soluble polymer can generally be added in an amount of 0.1 to 500 parts by weight based on 1 part by weight of the above-described azasteroid. It is preferably added in an amount of 0.2 to 300 parts by weight (more preferably 10 parts by weight, particularly preferably 6 parts by weight and most preferably 5 parts by weight) based on 1 part by weight of the azasteroid.

The above-described disintegrant can generally be added in an amount of 0.1 to 500 parts by weight based on 1 part by weight of the azasteroid. Preferably, it is added in an amount of 0.25 part by weight (more preferably 0.5 part by weight and most preferably 1 part by weight) to 300 parts by weight (preferably 10 parts by weight, particularly preferably 6 parts by weight and most preferably 5 parts by weight) based on 1 part by weight of the azasteroid.

The disintegrant can generally be added in an amount of 0.1 to 100 parts by weight based on 1 part by weight of the above-described water-soluble polymer. It is preferably added in an amount of 0.5 part by weight (more preferably 1 part by weight) to 10 parts by weight (preferably 5 parts by weight) based on 1 part by weight of the water-soluble polymer.

The water-soluble polymer and the disintegrant can generally be added in a total amount of 0.2 to 1000 parts by weight based on 1 part by weight of the azasteroid. Preferably,they are added in a total amount of 0.5 part by weight (more preferably 1 part by weight and particularly preferably 2 parts by weight) to 600 parts by weight (more preferably 20 parts by weight, particularly preferably 10 parts by weight, more particularly preferably 6 parts by weight and most preferably 5 parts by weight) based on 1 part by weight of the azasteroid.

The azasteroids contained as an active ingredient of the "formulation of 5α-reductase inhibitor for oral administration" according to the present invention, that is, N-[1-(4-methoxyphenyl)-1-methylethyl]-3-oxo-4-aza-5α-androst-1-ene-17β-carboxamide [the compound represented by the above formula (I)] and N-(1,1-dimethylethyl)-3-oxo-4-aza-5α-androst-1-ene-17β-carboxamide [the compound represented by the above formula (II)], are prepared by the processes described in Japanese Patent Application Kokai No. HEI 5-32693 and Japanese Patent Application Kokai No. SHO 60-222497 (Japanese Patent Application Kokoku No. SHO 63-65080), respectively.

The "composition" contained by the "formulation of a 5α-reductase inhibitor for oral administration" of the present invention is obtained by grinding a mixture containing the azasteroid, water-soluble polymer and disintegrant.

The grinding can be performed in a known manner, preferably with a grinding machine which allows continuous grinding for a long time. Examples of such a grinding machine include those using a medium such as a rod mill and a ball mill, and an oscillating ball mill and a medium-agitating type grinding machine are preferred.

Although the time required for grinding changes according to the tool or machine to be used for grinding, the kind of compound to be ground or the amount of mixture to be ground, grinding is generally carried out for one minute to 24 hours.

The "composition" so prepared can be administered as it is to the human body as a 5α-reductase inhibitor, and also in this case it exhibits a markedly improved dissolution property.

Alternatively, it can be administered as various dosage forms obtainable by adding one or more additives to the "composition" and formulating the resulting mixture in a manner known per se in the art. As the dosage form, solid forms generally known as oral formulations are employed. Examples include powders, granules, tablets and capsules.

For the preparation of powders, for example, the above-described "composition" is used as it is or mixed uniformly with one or more additives described later.

The granules can be prepared, for example, by dry granulation or wet granulation. In this case, it is possible to add, if necessary, one or more of the below-described additives to the "composition" before or after granulation.

The tablets can be prepared, for example, by directly tableting the "composition". Alternatively, they can be obtained by preparing granules from the "composition" and then tableting the resulting granules. In either case, one or more of the below-described additives can be added, if necessary, prior to tableting.

The capsules can be prepared, for example, by filling capsules with the "composition" as it is. Alternatively, they can be obtained by preparing granules as described above in advance and then filling capsules with them. In either case, one or more of the below-described additives can be added, if necessary, prior to filling.

Examples of the additive employed for the preparation of a pharmaceutical formulation from the "composition" include excipients, binders, disintegrants, colorants, taste masking agents/smell masking agents and lubricants.

As examples of the excipient, there are included kaolin, casein, powdered glycyrrhiza, agar, light anhydrous silicic acid, natural aluminium silicate, synthetic aluminium silicate, synthetic magnesium silicate, silicic anhydride, magnesium silicate, wheat flour, heavy magnesium oxide, aluminium hydroxide, magnesium carbonate co-precipitate, dried aluminium hydroxide gel, gypsum, exsiccated gypsum, gelatin, microcrystalline cellulose, D-sorbitol, calcium carbonate, precipitated calcium carbonate, magnesium carbonate, sodium bicarbonate, talc, dextrin, starch, lactose, calcium lactate, sucrose, glucose, pectin, malt extract, D-mannitol, magnesium metasilicate aluminate, aluminium monostearate, purified lanolin, dibasic calcium phosphate and dibasic sodium phosphate. Preferred are lactose, D-mannitol, sucrose, purified saccharose and crystalline cellulose.

As examples of the binder, there are included sugar syrup, starch syrup, gum arabic, gum arabic powder, ethanol, ethylcellulose, casein sodium, carmellose, carmellose calcium, KANBAIKO, glycerin, cellulose acetate phthalate, stearic acid, purified water, gelatin, purified shellac, white shellac, dextrin, starch, tragacanth, powdered tragacanth, honey, microcrystalline cellulose, hydroxycellulose, hydroxypropylcellulose, hydroxypropyl starch, hydroxypropylmethyl cellulose, hydroxymethylcellulose, sodium polyphosphate, rice flour, methylcellulose, methylcellulose sodium and polyvinylpyrrolidone.

Examples of the disintegrant include those exemplified above as "disintegrant".

Examples of the colorant include caramel, iron sesquioxide, tar colorants used for pharmaceuticals.

Examples of the taste masking agent/smell masking agent include ordinarily employed sweeteners, acidifiers and flavors.

As examples of the lubricant, there are included carnauba wax, light anhydrous silicic acid, synthetic aluminium silicate, natural aluminium silicate, synthetic magnesium silicate, hydrogenated oil, hydrogenated vegetable oil derivatives (for example, Sterotex HM), sesame oil, white beeswax, titanium oxide, dried aluminium hydroxide gel, stearic acid, calcium stearate, magnesium stearate, talc, dibasic calcium phosphate and sodium laurylsulfate.

It is also possible to add the above-exemplified additives to the mixture before grinding insofar as they do not adversely affect the grinding and the dissolution property of the azasteroid and then grind the resulting mixture, thereby preparing the above-described "composition".

Although the dose of a "formulation of a 5α-reductase inhibitor for oral administration of the invention" will change according to the symptoms and age of the patient, it is desired to administer, once or in several portions, the formulation which has been adjusted to contain the azasteroid in an amount ranging from 0.1 mg (preferably 0.5 mg) as the lower limit to 100 mg (preferably 50 mg, most preferably 20 mg) as the upper limit per day.

### Best Mode for performing the Invention

The invention will hereinafter be described more specifically by the following examples, comparative examples and tests.

### Example 1

In a mortar, 1.0 g of N-[1-(4-methoxyphenyl)-1-methylethyl]-3-oxo-4-aza-5α-androst-1-ene-17β-carboxamide (which will hereinafter be called "Compound I"), 0.5 g of hydroxypropylmethylcellulose (which will hereinafter be abbreviated as "HPMC") and 2 g of crospovidone ("Kollidon CL", trade name; product of BASF Japan) were mixed uniformly, followed by grinding for 30 minutes in a VIBRATING SAMPLE MILL model TI-100 (CMT Co., Ltd.) having a rod therein.

### Example 2

In a mortar, 1.0 g of Compound I, 0.5 g of HPMC and 2 g of sodium carboxymethyl starch ("Explotab", trade name; product of Kimura Sangyo Co., Ltd.) were mixed uniformly, followed by grinding for 30 minutes in a VIBRATING SAMPLE MILL model TI-100 having a rod therein.

### Example 3

In a mortar, 1.0 g of Compound I, 0.5 g of HPMC and 2 g of croscarmellose sodium ("Ac-Di-Sol", trade name; product of Asahi Chemical Industry Co., Ltd.) were mixed uniformly, followed by grinding for 30 minutes in a VIBRATING SAMPLE MILL model TI-100 having a rod therein.

### Example 4

Compound I (1170 g), 286 g of HPMC, 286 g of carmellose calcium and 258 g of crystalline cellulose were mixed. In a DYNAMIC MILL Type 10 (Mitsui Miike Engineering Corporation), the resulting mixture was charged and ground at a rate of 60 g/min.

### Example 5

Compound I (2 kg), 3.9 kg of carmellose calcium, 0.5 kg of HPMC and 0.4 kg of crystalline cellulose were mixed. In a DYNAMIC MILL Type 10 (Mitsui Miike Engineering Corporation), the resulting mixture was charged and ground at a rate of 120 g/min.

### Example 6

In a mortar, 1 g of N-(1,1-dimethylethyl)-3-oxo-4-aza-5α-androst-1-ene-17β-carboxamide (which will hereinafter be called "Compound II"), 3 g of HPMC and 2 g of Kollidon CL were mixed uniformly, followed by grinding for 30 minutes in a VIBRATING SAMPLE MILL model TI-100 having a rod therein.

### Comparative Example 1

In a VIBRATING SAMPLE MILL model TI-100 having a rod therein, 5 g of Compound I in the form of crystals were charged, followed by grinding for 30 minutes.

### Comparative Example 2

1170 g of Compound I, 286 g of HPMC, 286 g of carmellose calcium and 258 g of crystalline cellulose were mixed.

### Comparative Example 3

10 mg of Compound I were dissolved in 20 ml of polyethylene glycol 400.

### Test 1: Dissolution test

Amounts of the compositions obtained in Examples 1 to 4, the powders obtained in Comparative Example 1 and the mixture obtained in Comparative Example 2, each corresponding to 10 mg of Compound I were weighed. They were subjected to a dissolution test in accordance with the method 2 (paddle method) described in the Dissolution Test defined in the Pharmacopoeia of Japan (the 12th edition).

In the case of the compositions obtained in Examples 1 to 3 and the powders obtained in Comparative Example 1, 900 ml of the second test fluid as described in the Disintegration Test defined in the Pharmacopoeia of Japan (the 12th edition) were used as the solution for dissolution. The test was conducted at a paddle rotational frequency of 200 rpm. In the case of the composition obtained in Example 4 and the mixture obtained in Comparative Example 2, water was used as a solution for dissolution and the test was conducted at a paddle rotational rate of 200 rpm. Results are shown in Tables 1 and 2.

**Table 1:**

| Results of Dissolution Test 1 | | | | |
|---|---|---|---|---|
| | Example 1 | Example 2 | Example 3 | Comparative Example 1 |
| D(%) 30 min | 94 | 91 | 90 | 5 |
| D(%) 30 min: Dissolution ratio 30 minutes after the beginning of the dissolution test. | | | | |

**Test 2:**

| Results of Dissolution Test 2 | | |
|---|---|---|
| | Example 4 | Comparative Example 2 |
| D(%) 30 min | 92 | 7 |
| D(%) 30 min: Dissolution ratio 30 minutes after the beginning of the dissolution test. | | |

As is apparent from Tables 1 and 2, the dissolution property of Compound I was improved enough for practical use by grinding a mixture of Compound I, a water-soluble polymer and a disintegrant. Thus, the formulation of a 5α-reductase inhibitor for oral administration of the invention is markedly useful, because it is not necessary to incorporate an excess amount of an active ingredient in one dosage unit and because, in addition, fluctuations in the absorption ratio among individual living bodies administered can be suppressed to the minimum.

### Test 2: Change of concentration of Compound I in blood

An amount of the composition obtained in Example 5 corresponding to 10 mg of Compound I was suspended in 20 ml of water to prepare a suspension. The resulting suspension and the solution obtained in Comparative Example 3 were respectively orally administered to beagles. Blood samples were obtained from the upper portion of its forearm with the lapse of time and concentration (ng/ml) of Compound I in the blood was measured. Results are shown in Table 3.

**Table 3**

| Change of concentration (ng/ml) of Compound I in blood | | |
|---|---|---|
| Time (hr) | Suspension of the composition obtained in Ex. 5 | Solution of Comparative Ex. 2 |
| 0 | 0 | 0 |
| 0.25 | 391 | 369 |
| 0.5 | 589 | 635 |
| 1 | 794 | 800 |
| 1.5 | 873 | 743 |
| 2 | 856 | 671 |
| 3 | 801 | 516 |
| 4 | 596 | 410 |
| 6 | 358 | 270 |
| 8 | 187 | 175 |

From Table 3, it has been found that the formulation of a 5α-reductase inhibitor for oral administration of the present invention has a bioavailability equivalent to the solution of Compound I.

### Test 3: Improvement in dissolution property by grinding

An amount of the composition obtained in Example 6 corresponding to 100 mg of Compound II was weighed and a dissolution test for it was conducted in accordance with the method 2 (paddle method) described in the Dissolution Test of the Pharmacopoeia of Japan (the 12th edition). In the test, 900 ml of the second test fluid as described in the Disintegration Test of the Pharmacopoeia of Japan (the 12th edition) were used as the solution for dissolution and the test was conducted at a paddle rotational rate of 200 rpm.

**Table 4**

| Improvement in dissolution property by grinding | | |
|---|---|---|
| | Before grinding | After grinding |
| D(%) min | 36 | 78 |

As is apparent from Table 4, the dissolution property of Compound II was markedly improved by grinding the mixture.

## Claims

1. A formulation of a 5α-reductase inhibitor for oral administration which comprises a composition obtained by grinding a mixture containing one azasteroid selected from the group (A) shown below, a water-soluble polymer and a disintegrant:
Group (A)
N-[1-(4-methoxyphenyl)-1-methylethyl]-3-oxo-4-aza-5α-androst-1-ene-17β-carboxamide; and
N-(1,1-dimethylethyl)-3-oxo-4-aza-5α-androst-1-ene-17β-carboxamide.

2. A formulation of a 5α-reductase inhibitor for oral administration according to claim 1, wherein said one azasteroid selected from the group (A) is N-[1-(4-methoxyphenyl)-1-methylethyl]-3-oxo-4-aza-5α-androst-1-ene-17β-carboxamide.

3. A formulation of a 5α-reductase inhibitor for oral administration according to claim 1 or 2, wherein said water-soluble polymer is a water-soluble cellulose derivative or polyalkenyl pyrrolidone derivative.

4. A formulation of a 5α-reductase inhibitor for oral administration according to claim 1 or 2, wherein said water-soluble polymer is hydroxypropylmethyl cellulose, hydroxypropylcellulose or polyvinylpyrrolidone.

5. A formulation of a 5α-reductase inhibitor for oral administration according to any one of claims 1 to 4, wherein said disintegrant is sodium carboxymethyl starch, crospovidone, carmellose, carmellose calcium, croscarmellose sodium, low-substituted hydroxypropylcellulose or crystalline cellulose.

6. A formulation of a 5α-reductase inhibitor for oral administration according to any one of claims 1 to 4, wherein said disintegrant is sodium carboxymethyl starch, crospovidone, carmellose, carmellose calcium or croscarmellose sodium.

7. A formulation of a 5α-reductase inhibitor for oral administration according to any one of claims 1 to 6, wherein said water soluble polymer is added in an amount of 0.1 to 500 parts by weight based on 1 part by weight of said azasteroid.

8. A formulation of a 5α-reductase inhibitor for oral administration according to any one of claims 1 to 7, wherein said disintegrant is added in an amount of 0.1 to 500 parts by weight based on 1 part by weight of said azasteroid.

9. A formulation of a 5α-reductase inhibitor for oral administration according to any one of claims 1 to 8, wherein said disintegrant is added in an amount of 0.5 to 10 parts by weight based on 1 part by weight of said water-soluble polymer.

10. A process for the preparation of a formulation of a 5α-reductase inhibitor for oral administration, which comprises grinding a mixture containing one azasteroid selected from the group (A) shown below, a water-soluble polymer and a disintegrant:
Group A
N-[1-(4-methoxyphenyl)-1-methylethyl]-3-oxo-4-aza-5α-androst-1-ene-17β-carboxamide; and
N-(1,1-dimethylethyl)-3-oxo-4-aza-5α-androst-1-ene-17β-carboxamide.

11. A process according to claim 10, wherein said one azasteroid selected from the group (A) is N-[1-(4-methoxyphenyl)-1-methylethyl] -3-oxo-4-aza-5α-androst-1-ene-17β-carboxamide.

12. A process according to claim 10 or 11, wherein said water-soluble polymer is a water-soluble cellulose derivative or a poly(alkenylpyrrolidone) derivative.

13. A process according to claim 10 or 11, wherein said water-soluble polymer is hydroxypropylmethyl cellulose, hydroxypropylcellulose or poly (vinylpyrrolidone).

14. A process according to any one of claims 10 to 13, wherein said disintegrant is sodium carboxymethyl starch, crospovidone, carmellose, carmellose calcium, croscarmellose sodium, low-substituted hydroxypropylcellulose or crystalline cellulose.

15. A process according to any one of claims 10 to 13, wherein said disintegrant is sodium carboxymethyl starch, crospovidone, carmellose, carmellose calcium or croscarmellose sodium.

16. A process according to any one of claims 10 to 15, wherein said water soluble polymer is added in an amount of 0.1 to 500 parts by weight based on 1 part by weight of said azasteroid.

17. A process according to any one of claims 10 to 16, wherein said disintegrant is added in an amount of 0.1 to 500 parts by weight based on 1 part by weight of said azasteroid.

18. A process according to any one of claims 10 to 17, wherein said disintegrant is added in an amount of 0.5 to 10 parts by weight based on 1 part by weight of said water-soluble polymer.

## Patentansprüche

1. 5α-Reductaseinhibitor-Zubereitung zur oralen Verabreichung, welche eine Zusammensetzung umfasst, die durch Mahlen eines Gemisches erhalten wird, welches ein aus der nachstehend gezeigten Gruppe (A) ausgewähltes Azastereoid, ein wasserlösliches Polymer und ein Zerfallhilfsmittel enthält:
Gruppe (A)
N-[1-(4-Methoxyphenyl)-1-methylethyl]-3-oxo-4-aza-5α-androst-1-en-17β-carboxamid und
N-[1,1-Dimethylethyl)-3-oxo-4-aza-5α-androst-1-en-17β-carboxamid.

2. 5α-Reductaseinhibitor-Zubereitung für die orale Verabreichung nach Anspruch 1, wobei das aus Gruppe (A) ausgewählte Azastereoid N-[1-(4-Methoxyphenyl)-1-methylethyl]-3-oxo-4-aza-5α-androst-1-en-17β-carboxamid ist.

3. 5α-Reductaseinhibitor-Zubereitung für die orale Verabreichung nach Anspruch 1 oder 2, wobei das wasserlösliche Polymer ein wasserlösliches Cellulosederivat oder Polyalkenylpyrrolidonderivat ist.

4. 5α-Reductaseinhibitor-Zubereitung für die orale Verabreichung nach Anspruch 1 oder 2, wobei das wasserlösliche Polymer Hydroxypropylmethylcellulose, Hydroxypropylcellulose oder Polyvinylpyrrolidon ist.

5. 5α-Reductaseinhibitor-Zubereitung für die orale Verabreichung nach einem der Ansprüche 1 bis 4, wobei das Zerfallhilfsmittel Natriumcarboxymethylstärke, Crospovidon, Carmellose, Carmellose-Calcium, Croscarmellose-Natrium, niedrig substituierte Hydroxypropylcellulose oder kristalline Cellulose ist.

6. 5α-Reductaseinhibitor-Zubereitung für die orale Verabreichung nach einem der Ansprüche 1 bis 4, wobei das Zerfallhilfsmittel Natrium-Carboxymethylstärke, Crospovidon, Carmellose, Carmellose-Calcium oder Croscarmellose-Natrium ist.

7. 5α-Reductaseinhibitor-Zubereitung für die orale Verabreichung nach einem der Ansprüche 1 bis 6, wobei das wasserlösliche Polymer in einer Menge von 0,1 bis 500 Gewichtsteilen, bezogen auf 1 Gewichtsteil des Azastereoids, zugesetzt wird.

8. 5α-Reductaseinhibitor-Zubereitung für die orale Verabreichung nach einem der Ansprüche 1 bis 7, wobei das Zerfallhilfsmittel in einer Menge von 0,1 bis 500 Gewichtsteilen, bezogen auf 1 Gewichtsteil des Azastereoids, zugesetzt wird.

9. 5α-Reductaseinhibitor-Zubereitung für die orale Verabreichung nach einem der Ansprüche 1 bis 8, wobei das Zerfallhilfsmittel in einer Menge von 0,5 bis 10 Gewichtsteilen, bezogen auf 1 Gewichtsteil des wasserlöslichen Polymers, zugesetzt wird.

10. Verfahren zur Herstellung einer 5α-Reductaseinhibitor-Zubereitung für die orale Verabreichung, welches das Mahlen eines Gemisches umfasst, welches ein aus der nachstehend gezeigten Gruppe (A) ausgewähltes Azastereoid, ein wasserlösliches Polymer und ein Zerfallhilfsmittel enthält:
Gruppe A
N-[1-(4-Methoxyphenyl)-1-methylethyl]-3-oxo-4-aza-5α-androst-1-en-17ß-carboxamid und
N-[1,1-Dimethylethyl)-3-oxo-4-aza-5a-androst-1-en-17β-carboxamid.

11. Verfahren nach Anspruch 10, wobei das aus der Gruppe (A) ausgewählte Azastereoid N-[1-(4-Methoxyphenyl)-1-methylethyl]-3-oxo-4-aza-5α-androst-1-en-17β-carboxamid ist.

12. Verfahren nach Anspruch 10 oder 11, wobei das wasserlösliche Polymer ein wasserlösliches Cellulosederivat oder Poly(alkenylpyrrolidon)derivat ist.

13. Verfahren nach Anspruch 10 oder 11, wobei das wasserlösliche Polymer Hydroxypropylmethylcellulose, Hydroxypropylcellulose oder Polyvinylpyrrolidon ist.

14. Verfahren nach einem der Ansprüche 10 bis 13, wobei das Zerfallhilsmittel Natrium-Carboxymethylstärke, Crospovidon, Carmellose, Carmellose-Calcium, Croscarmellose-Natrium, niedrig substituierte Hydroxypropylcellulose oder kristalline Cellulose ist.

15. Verfahren nach einem der Ansprüche 10 bis 13, wobei das Zerfallhilfsmittel Natrium-Carboxymethylstärke, Crospovidon, Carmellose, Carmellose-Calcium oder Croscarmellose-Natrium ist.

16. Verfahren nach einem der Ansprüche 10 bis 15, wobei das wasserlösliche Polymer in einer Menge von 0,1 bis 500 Gewichtsteilen, bezogen auf 1 Gewichtsteil des Azastereoids, zugesetzt wird.

17. Verfahren nach einem der Ansprüche 10 bis 16, wobei das Zerfallhilfsmittel in einer Menge von 0,1 bis 500 Gewichtsteilen, bezogen auf 1 Gewichtsteil des Azastereoids, zugesetzt wird.

18. Verfahren nach einem der Ansprüche 10 bis 16, wobei das Zerfallhilfsmittel in einer Menge von 0,5 bis 10 Gewichtsteilen, bezogen auf 1 Gewichtsteil des wasserlöslichen Polymers, zugesetzt wird.

## Revendications

1. Formulation d'un inhibiteur de 5α-réductase pour l'administration orale qui comprend une composition obtenue en broyant un mélange contenant un azastéroide choisi dans le groupe (A) représenté ci-dessous, un polymère hydrosoluble et un agent désintégrant :
Groupe (A)
N-(1-(4-méthoxyphényl)-1-méthyléthyl)-3-oxo-4-aza-5α-androst-1-ène-17β-carboxamide, et
N-(1,1-diméthyléthyl)-3-oxo-4-aza-5α-androst-1-ène-17β-carboxamide.

2. Formulation d'un inhibiteur de 5α-réductase pour l'administration orale selon la revendication 1, **caractérisé en ce que** ledit azastéroïde choisi dans le groupe (A) est le N-[1-(4-méthoxyphényl)-1-méthyléthyl]-3-oxo-4-aza-5α-androst-1-ène-17β-carboxamide.

3. Formulation d'un inhibiteur de 5α-réductase pour l'administration orale selon la revendication 1 ou 2, **caractérisé en ce que** ledit polymère hydrosoluble est un dérivé de cellulose ou un dérivé de polyalcénylpyrrolidone hydrosoluble.

4. Formulation d'un inhibiteur de 5α-réductase pour l'administration orale selon la revendication 1 ou 2, **caractérisé en ce que** ledit polymère hydrosoluble est l'hydroxypropylméthylcellulose, l'hydroxypropylcellulose ou la polyvinylpyrrolidone.

5. Formulation d'un inhibiteur de 5α-réductase pour l'administration orale selon l'une quelconque des revendications 1 à 4, **caractérisé en ce que** ledit agent désintégrant est l'amidon carboxyméthylé sodique, la crospovidone, la carmellose, la carmellose calcique, la croscarmellose sodique, l'hydroxypropylcellulose faiblement substituée ou la cellulose cristalline.

6. Formulation d'un inhibiteur de 5α-réductase pour l'administration orale selon l'une quelconque des revendications 1 à 4, **caractérisé en ce que** ledit agent de désintégration est l'amidon carboxyméthylé de sodium, la crospovidone, la carmellose, la carmellose calcique ou la croscarmellose sodique.

7. Formulation d'un inhibiteur de 5α-réductase pour l'administration orale selon l'une quelconque des revendications 1 à 6, **caractérisé en ce que** ledit polymère hydrosoluble est ajouté en une quantité de 0,1 à 500 parties en poids rapporté à 1 partie en poids dudit azastéroïde.

8. Formulation d'un inhibiteur de 5α-réductase pour l'administration orale selon l'une quelconque des revendications 1 à 7, **caractérisé en ce que** ledit agent désintégrant est ajouté en une quantité de 0,1 à 500 parties en poids rapporté à 1 partie en poids dudit azastéroïde.

9. Formulation d'un inhibiteur de 5α-réductase pour l'administration orale selon l'une quelconque des revendications 1 à 8, **caractérisé en ce que** ledit agent désintégrant est ajouté en une quantité de 0,5 à 10 parties en poids rapporté à 1 partie en poids dudit polymère hydrosoluble.

10. Procédé de préparation d'une formulation d'un inhibiteur de 5α-réductase pour l'administration orale qui comprend le broyage d'un mélange contenant un azastéroïde choisi dans le groupe (A) représenté ci-dessous, un polymère hydrosoluble et un agent désintégrant :
Groupe (A)
N-[1-(4-méthoxyphényl)-1-méthyléthyl]-3-oxo-4-aza-5α-androst-1-ène-17β-carboxamide, et
N-(1,1-diméthylélhyl)-3-oxo-4-aza-5α-androst-1-ène-17β-carboxamide.

11. Procédé selon la revendication 10, **caractérisé en ce qu'**un azastéroïde choisi dans le groupe (A) est le N-[1-(4-méthoxyphényl)-1-méthyléthyl]-3-oxo-4-aza-5α-androst-1-ène-17β-carboxamide.

12. Procédé selon la revendication 10 ou 11, **caractérisé en ce que** ledit polymère hydrosoluble est un dérivé de cellulose ou un dérivé de poly(alcénylpyrrolidone) hydrosoluble.

13. Procédé selon la revendication 10 ou 11, **caractérisé en ce que** ledit polymère hydrosoluble est l'hydroxypropylméthylcellulose, l'hydroxypropylcellulose ou la poly(vinylpyrrolidone).

14. Procédé selon l'une quelconque des revendications 10 à 13, **caractérisé en ce que** ledit agent désintégrant est l'amidon carboxyméthylé sodique, la crospovidone, la carmellose, la carmellose calcique, la croscarmellose sodique, l'hydroxypropylcellulose faiblement substituée ou la cellulose cristalline.

15. Procédé selon l'une quelconque des revendications 10 à 13, **caractérisé en ce que** ledit agent désintégrant est l'amidon carboxyméthylé sodique, la crospovidone, la carmellose, la carmellose calcique ou la croscarmellose sodique.

16. Procédé selon l'une quelconque des revendications 10 à 15, **caractérisé en ce que** ledit polymère hydrosoluble est ajouté en une quantité de 0,1 à 500 parties en poids rapporté à 1 partie en poids dudit azastéroïde.

17. Procédé selon l'une quelconque des revendications 10 à 16, **caractérisé en ce que** ledit agent désintégrant est ajouté en une quantité de 0,1 à 500 paries en poids rapporté à 1 partie en poids dudit azastéroïde.

18. Procédé selon l'une quelconque des revendications 10 à 17, **caractérisé en ce que** ledit agent désintégrant est ajouté en une quantité de 0,5 à 10 parties en poids rapporté à 1 partie en poids dudit polymère hydrosoluble.
